# EUROPÄISCHE PATENTSCHRIFT

(11) **EP 1 785 124 B1**
(45) Veröffentlichungstag und Bekanntmachung des Hinweises auf die Patenterteilung: **22.08.2012**
(21) Anmeldenummer: 06123442.3
(22) Anmeldetag: 03.11.2006
(51) Int. Cl.: A61G 13/12, A61G 13/08

(54) **Polsterelement für eine Patientenliegefläche eines Operationstisches**
Padding element for a patient supporting surface on an operation table
Element de rembourrage pour supporter un patient sur une table d'opération

(30) Priorität: 10.11.2005 DE 102005053755
(43) Veröffentlichungstag der Anmeldung: 16.05.2007
(73) Patentinhaber: MAQUET GmbH & Co. KG, 76437 Rastatt (DE)
(72) Erfinder: Kobuß, Matthias, 76571, Gaggenau (DE); Katzenstein, Bernhard, 76532, Baden-Baden (DE); Olszewski, Jan Donat, 76437, Rastatt (DE)
(74) Vertreter: Schaumburg, Thoenes, Thurn, Landskron, Eckert

(56) Entgegenhaltungen:
- FR-A- 2 484 248
- US-B1- 6 493 417

## Beschreibung

Die vorliegende Erfindung betrifft ein Polsterelement für eine Patientenliegefläche, beispielsweise für eine Liegefläche eines Operationstisches. Operationstische haben typischerweise eine Liegefläche, die aus mehreren relativ zueinander verstellbaren Segmenten besteht. Auf den unterschiedlichen Segmenten sind Polsterelemente befestigt, auf denen der Patient gelagert ist. Üblicherweise bestehen die Polsterelemente aus formstabilem Integralschaumstoff, mit dem sich geeignete Formen und Strukturen leicht herstellen lassen. Der Integralschaumstoff hat jedoch den Nachteil, dass er verhältnismäßig hart ist, so dass ein Patient von einer längeren Lagerung auf einem derartigen Polsterelement Druckstellen bekommen kann.

Im Prinzip kann zusätzlich zu dem Integralschaumpolster ein weicheres Polster verwendet werden, welches mit Bändern oder Klettverschlüssen an dem Integralschaumpolster befestigt ist. Derartige Befestigungsmittel wie Gurte, Bänder oder Klettverbindungen lassen sich jedoch schlecht reinigen und stellen somit ein hygienisches Problem dar. Außerdem ist die Befestigung und das Lösen zusätzlicher weicher Polster umständlich und daher zeitraubend.

Aus der US-B1-6493417 ist eine Patientenlagerfläche für CT-Geräte und dergleichen bekannt, umfassend eine Bodenplatte und eine auf dieser mit Hilfe von Klettverschlüssen befestigbare Polstermatte. Die Polstermatte hat auf ihrer der Bodenplatte zugewandten Unterseite eine Ausnehmung, in die ein Referenzkörper eingelegt werden kann.

Der Erfindung liegt die Aufgabe zugrunde, ein Polsterelement der eingangs genannten Art anzugeben, das sich leicht handhaben und reinigen lässt und gleichzeitig eine weiche Lagerung des Patienten ermöglicht.

Diese Aufgabe wird durch ein Polsterelement gemäß dem Anspruch 1 gelöst.

Bei dem erfindungsgemäßen Polsterelement ist somit ein Polsterteil vorgesehen, welches zumindest teilweise aus Weichschaumstoff besteht und somit eine weiche Lagerung eines Patienten gestattet, durch die Druckstellen vermieden werden können. Das weichere Polsterteil erhält durch die formstabilen Seitenteile zwischen die es gesetzt werden kann, seitlichen Halt. Gleichzeitig wird das Polsterteil durch den Eingriff des mindestens einen vorstehenden Abschnittes in seine zugehörige Ausnehmung in dieser Lage festgehalten, ohne dass zusätzliche Befestigungsmittel wie Klettverbindungen, Bänder oder Gurte benötigt werden. Dadurch lässt sich das Polsterelement leicht und gründlich reinigen und einfach handhaben.

Vorzugsweise bestehen die Seitenteile aus Integralschaum, insbesondere PUR-Integralschaum,

In einer bevorzugten Ausführungsform werden die vorstehenden Abschnitte durch konvexe, insbesondere teilsphärische Noppen gebildet, die von einer nach innen gerichteten Seite eines jeden Seitenteils abstehen. Bei dieser Ausführungsform kann das Polsterteil leicht von oben zwischen die beiden Seitenteile gedrückt werden, wobei die Noppen an der jeweiligen Außenseite des Polsterteils entlang gleiten, welches dabei durch die vorstehenden Noppen etwas komprimiert wird, bis die Noppen in die zugehörigen Ausnehmungen eingreifen und das Polsterteil seine ursprüngliche Form wieder annimmt, in der es durch die Noppen in seiner Position festgehalten wird.

Das Polsterteil ist vorzugsweise von einer wasserundurchlässigen Hülle umgeben und ist somit abwaschbar. Es umfasst vorzugsweise eine untere Lage aus EPDM und eine obere Lage aus Weichschaumstoff. Die obere Lage aus Weichschaumstoff ermöglicht eine weiche Lagerung des Patienten. Die untere Lage aus dem etwas härteren EPDM kann die vorstehenden Abschnitte der Seitenteile untergreifen, wodurch das Polsterteil im Gebrauch sicherer gehalten wird.

Die Bodenplatte besteht vorzugsweise aus einem röntgenstrahlendurchlässigen Material, insbesondere aus Kunststoff, so dass der Patient auf dem Operationstisch aufliegend durchleuchtet werden kann.

Das Polsterelement ist vorzugsweise geeignet, auf einem Segment einer Liegefläche eines Operationstisches angeordnet zu werden, welches gelenkig mit einem weiteren Segment der Liegefläche verbunden ist. Dabei ist an mindestens einem der Seitenteile an mindestens einem seiner Längsenden ein Formabschnitt so ausgebildet, dass er ein Gelenk zwischen den beiden Segmenten zumindest teilweise überdeckt. Dadurch wird verhindert, dass ein unter Verwendung des Polsterelements gelagerter Patient mit dem Gelenk in Kontakt gerät. Ein solcher Formabschnitt erfordert eine komplexe Struktur, da er die unterschiedlichen Stellungen des Gelenkes gestatten muss. Eine derartige komplexe Struktur lässt sich jedoch mit lntegralschaum hervorragend herstellen.

Schließlich betrifft die Erfindung weiterhin einen Operationstisch mit einer Liegefläche, die mehrere relativ zueinander verstellbare Segmente umfasst, wobei auf mindestens einem der Segmente ein Polsterelement nach einer der oben erwähnten Weiterbildungen angeordnet ist.

Zum besseren Verständnis der vorliegenden Erfindung wird im folgenden auf das in den Zeichnungen dargestellte bevorzugte Ausführungsbeispiel bezug genommen, welches anhand spezifischer Terminologie beschrieben ist. Es sei jedoch darauf hingewiesen, dass der Schutzumfang der Erfindung dadurch nicht eingeschränkt werden soll, da derartige Veränderungen und weitere Modifizierungen an der gezeigten Einrichtung sowie derartige weitere Anwendungen der Erfindung, wie sie darin aufgezeigt sind, als übliches derzeitiges und künftiges Fachwissen eines zuständigen Fachmannes angesehen werden. Die Figuren zeigen ein Ausführungsbeispiel der Erfindung, nämlich
- Figur 1: eine Draufsicht auf ein Polsterelement nach einer Weiterbildung der Erfindung,
- Figur 2: eine Querschnittsansicht entlang der Linie A-A von Figur 1,
- Figur 3: eine perspektivische Ansicht des Polsterelements,
- Figur 4: eine Vorderansicht des Polsterelements von Figuren 1 und 2, bei dem das Polsterteil entfernt wurde,
- Figur 5: eine Draufsicht auf das Polsterelement von Figuren 1 und 2, bei dem das Polsterteil entfernt wurde und
- Figur 6: eine der Figur 2 entsprechende Querschnittsansicht durch eine abgewandelte Ausführungsform des erfindungsgemäßen Polsterelementes.

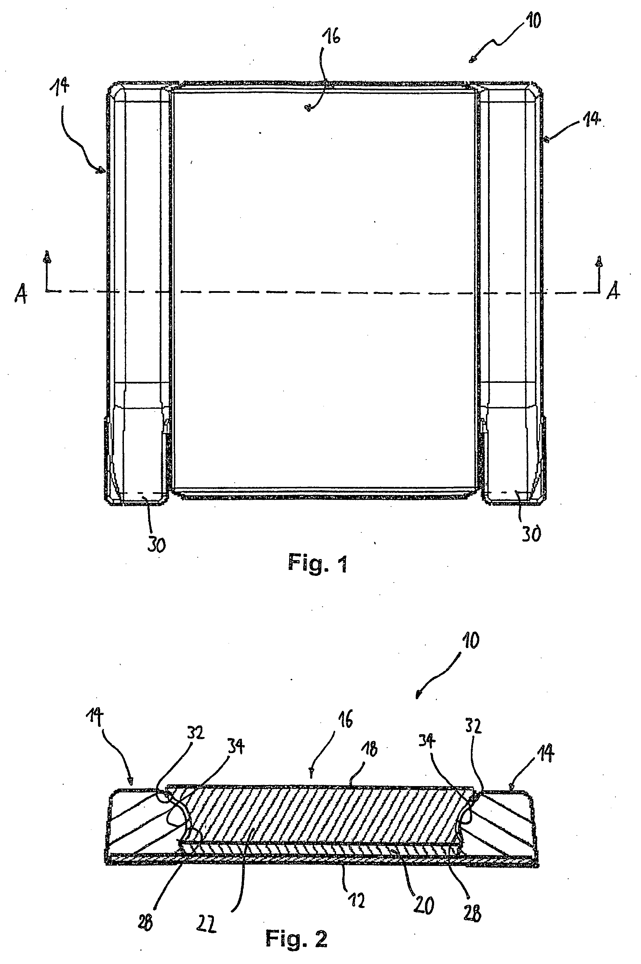

Figur 1 zeigt eine Draufsicht auf ein Polsterelement 10 nach einer Weiterbildung der Erfindung, und Figur 2 zeigt einen Querschnitt durch das Polsterelement 10 entlang der Linie A-A von Figur 1.

Das Polsterelement 10 ist dazu bestimmt, auf einem Segment einer Liegefläche eines Operationstisches (nicht gezeigt) angeordnet zu werden. Wie in Figur 1 und Figur 2 gezeigt ist, umfasst das Polsterelement 10 eine Bodenplatte 12, an deren Längsrändern Seitenteile 14 aus formstabilem Schaumstoff angeordnet sind. Dabei werden hier als Längsränder die Ränder der Bodenplatte 12 bezeichnet, die in Längsrichtung der Liegefläche des Operationstisches angeordnet sind, wenn das Polsterelement 10 auf dem Operationstisch angeordnet ist.

Die Bodenplatte 12 besteht aus röntgenstrahldurchlässigen Kunststoff. Somit können durch das Polsterelement 10 hindurch Röntgenaufnahmen eines darauf gelagerten Patienten gemacht werden.

Das Polsterelement 10 umfasst ferner ein Polsterteil 16, welches auf der Bodenplatte 12 aufliegend zwischen den länglichen Seitenteilen 14 angeordnet ist. Das Polsterteil 16 ist vollständig von einer abwaschbaren, wasserundurchlässigen Hülle 18 umgeben. Es enthält einen Kern, der aus einer unteren Lage 20 aus Ethylen-Propylen-Terpolymerschaum (EPDM-Schaum) und einer oberen Lage 22 aus Weichschaumstoff besteht. Die Hülle 18 kann zumindest an ihrer Außenoberfläche elektrisch leitfähig sein.

Das Polsterteil 16 kann auf die Bodenplatte 12 zwischen die Seitenteile 14 gesetzt werden, wie dies in Figuren 1 und 2 gezeigt ist, und wird in dieser Lage auf unten näher beschriebene Weise gehalten. Das Polsterteil 16 kann aus dieser Lage beispielsweise zum Reinigen entfernt werden.

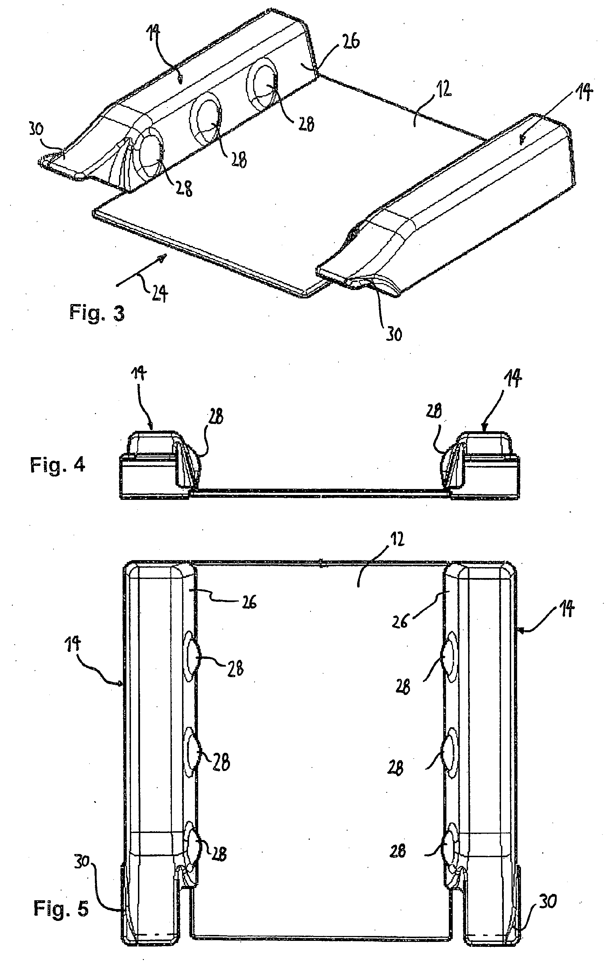

In Figuren 3 bis 5 ist das Polsterelement 10 mit entferntem Polsterteil 16 dargestellt. Dabei zeigt Figur 3 eine perspektivische Ansicht auf die Bodenplatte 12 und die beiden Seitenteile 14. Figur 4 zeigt eine Vorderansicht, d.h. eine Ansicht aus der Blickrichtung des Pfeils 24 von Figur 3, und Figur 5 eine Draufsicht auf die Bodenplatte 12 und die Seitenteile 14.

Wie oben bereits erwähnt wurde, bestehen die Seitenteile aus formstabilem Schaumstoff, aus dem sich die strukturierte Form der Seitenteile 14, die in den Figuren 3 bis 5 zu erkennen ist, gut herstellen läßt. Vorzugsweise bestehen die Seitenteile 14 aus einem Strukturschaumstoff oder Integralschaumstoff mit einer festen, nicht geschäumten Außenhaut, beispielsweise aus PUR-Integralschaum. Wie in den Figuren 3 bis 5 gezeigt ist, haben die Seitenteile 14 jeweils eine nach innen gerichtete Seite 26, an der jeweils drei vorstehende Noppen 28 ausgebildet sind. Die Noppen 28 haben eine konvexe, beispielsweise eine teilsphärische Gestalt. Die Seitenteile 14 können mit einem elektrisch leitfähigen Lack lackiert sein, so dass man in Verbindung mit der elektrisch leitfähigen Hülle 18 des Polsterteiles 16 eine vorschriftsmäßige elektrische Ableitung an der Oberfläche des Polsterelementes 10 erhält.

An einem der Längsenden der Seitenteile 14 ist jeweils ein Formabschnitt 30 ausgebildet. Der Formabschnitt 30 ist dazu bestimmt, ein Gelenk, welches zwischen zwei benachbarten Segmenten (nicht gezeigt) der Liegefläche eines Operationstisches angeordnet ist, teilweise zu überdecken. Dadurch wird sichergestellt, dass ein auf der Liegefläche gelagerter Patient nicht mit dem Gelenk in Kontakt kommt. Die komplexe, in den Figuren 3 bis 5 gezeigte Struktur der Seitenteile 14 lässt sich verhältnismäßig einfach aus Integralschaum herstellen.

Die Noppen 28 sind zum Festhalten des Polsterteils 16 bestimmt, wenn dieses wie in den Figuren 1 und 2 gezeigt auf der Bodenplatte 12 aufliegend zwischen den Seitenteilen 14 angeordnet ist. In dieser Lage greifen die Noppen 28 in zugehörige Ausnehmungen 34 des Polsterteils 16 ein, die an der Außenseite 32 des Polsterteils 16 ausgebildet sind (siehe Figur 2). Dadurch wird verhindert, dass das Polsterteil 16 relativ zur Bodenplatte 12 und zu den Seitenteilen 14 verrutscht. Dazu trägt insbesondere die untere Lage 22 des Polsters 16 bei, die aus EPDM-Schaum besteht und somit steifer als der viskoelastische Weichschaumstoff der oberen Lage 22 ist und die die Noppen 28 untergreift.

Das Polsterelement 10 aus den Figuren 1 bis 5 hat somit einen mittleren Liegeabschnitt, der durch das Weichpolsterteil 16 gebildet wird, und auf dem ein Patient lange gelagert werden kann, ohne dass sich an seinem Körper Druckstellen bilden. Gleichzeitig hat das Polsterelement die steiferen Seitenteile 14, durch die das Polsterteil in seiner vorbestimmten Lage festgehalten wird und die dem Polsterteil 16 seitlichen Halt verleihen. Ferner haben die Seitenteile 14 eine geeignete Struktur, um weitere Bestandteile des Operationstisches (nicht gezeigt), beispielsweise Längsholmen der Segmente oder Gelenke zwischen den Segmenten zu bedecken.

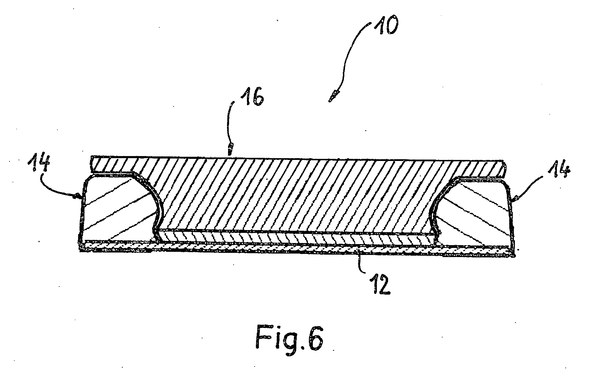

Bei einer in der Figur 6 dargestellten abgewandelten Ausführungsform hat das Polsterteil 16 einen T-förmigen Querschnitt, dessen T-Fuß zwischen den Seitenteilen 14 liegt und dessen T-Querarme die Seitenteile 14 überdecken, so dass das Polsterelement 10 in der Draufsicht eine geschlossene Oberfläche hat. Diese Lösung hat hygienische Vorteile, da Körperflüssigkeiten und andere Flüssigkeiten oder Verunreinigungen nicht in die Spalte zwischen den Seitenteilen 14 und dem Polsterteil 16 eindringen können.

Das Polsterteil 16 wird an dem Polsterelement 10 ohne Gurte, Klettverbindungen oder dergleichen, die sich schlecht reinigen lassen, sicher gehalten.

Obgleich in den Zeichnungen und in der vorhergehenden Beschreibung ein bevorzugtes Ausführungsbeispiel aufgezeigt und detailliert beschrieben ist, soll dies als rein beispielhaft und die Erfindung nicht einschränkend angesehen werden. Es wird darauf hingewiesen, dass nur das bevorzugte Ausführungsbeispiel dargestellt und beschrieben ist und sämtliche Veränderungen und Modifizierungen, die derzeit und künftig im Schutzumfang der Erfindung liegen, geschützt werden sollen.

## Patentansprüche

1. Polsterelement (10) für eine Patientenliegefläche mit
einer Bodenplatte (12), **gekennzeichnet durch**
zwei längliche Seitenteile (14) aus formstabilem Schaumstoff (22), welche entlang zweier gegenüberliegender Ränder der Bodenplatte (12) auf dieser angeordnet sind,
und ein Polsterteil (16), welches zumindest teilweise aus Weichschaumstoff besteht und herausnehmbar so zwischen die länglichen Seitenteile (14) auf die Bodenplatte (12) aufsetzbar ist, dass es mit seinen Außenseiten (32) zumindest teilweise an dem jeweils benachbarten Seitenteil (14) anliegt,
wobei zumindest an einem Seitenteil (14) mindestens ein vorstehender Abschnitt (28) so ausgebildet und angeordnet ist, dass er in eine zugehörige Ausnehmung (34) an der Außenseite (32) des Polsterteils (16) eingreift, wenn das Polsterteil (16) zwischen den Seitenteilen (14) angeordnet ist.

2. Polsterelement (10) nach Anspruch 1, bei dem die Seitenteile aus Integralschaum, insbesondere PUR-Integralschaum bestehen.

3. Polsterelement (10) nach Anspruch 1 oder 2, bei dem die vorstehenden Abschnitte durch konvexe, insbesondere teilsphärischen Noppen (28) gebildet werden, die von einer nach innen gerichteten Seite (26) eines jeden Seitenteils (24) abstehen.

4. Polsterelement (10) nach einem der vorhergehenden Ansprüche, bei dem das Polsterteil (16) mit einer wasserundurchlässigen Hülle (18) umgeben ist.

5. Polsterelement (10) nach Anspruch 4, **dadurch gekennzeichnet, dass** zumindest die Außenoberfläche der Hülle (18) elektrisch leitfähig ist.

6. Polsterelement (10) nach einem der vorhergehenden Ansprüche, bei dem das Polsterteil (16) eine untere Lage (20) aus EPDM-Schaumstoff und eine obere Lage (22) aus Weichschaumstoff umfasst.

7. Polsterelement (10) nach einem der vorhergehenden Ansprüche, **dadurch gekennzeichnet, dass** das Polsterteil (16) einen T-förmigen Querschnitt hat, dessen T-Fuß zwischen den Seitenteilen (14) liegt und dessen T-Querarme die Seitenteile (14) überdecken.

8. Polsterelement (10) nach einem der vorhergehenden Ansprüche, bei dem die Bodenplatte (12) aus einem röntgenstrahlendurchlässigen Material, insbesondere aus Kunststoff besteht.

9. Polsterelement (10) nach einem der vorhergehenden Ansprüche, das geeignet ist, auf einem Segment einer Liegefläche eines Operationstisches angeordnet zu werden, welches gelenkig mit einem weiteren Segment der Liegefläche verbunden ist.

10. Polsterelement (10) nach Anspruch 9, bei dem an mindestens einem der Seitenteile (14) an mindestens einem seiner Längsenden ein Formabschnitt (30) so ausgebildet ist, dass er ein Gelenk zwischen den genannten Segmenten zumindest teilweise überdeckt.

11. Operationstisch mit einer Liegefläche, die mehrere zueinander verstellbare Segmente umfasst, **dadurch gekennzeichnet, dass** auf mindestens einem der Segmente ein Polsterelement (10) nach einem der vorhergehenden Ansprüche angeordnet ist.

## Claims

1. An padding element (10) for a patient bed, with
a baseplate (12), **characterized by**
two elongate side parts (14) made of dimensionally stable foam (22) which are arranged on said baseplate (12) along two mutually opposite edges thereof,
and a padding part (16) which is made at least partially of soft foam and can be fitted removeably onto the baseplate (12) between the elongate side parts (14) such that it bears with its outer faces (32) at least partially against the respectively adjacent side part (14),
wherein at least one projecting portion (28) is formed and arranged at least on one side part (14) such that the said portion engages into an associated recess (34) on the outer face (32) of the padding part (16) when the padding part (16) is arranged between the side parts (14).

2. The padding element (10) according to Claim 1, in which the side parts are made of integral foam, in particular of PUR integral foam.

3. The padding element (10) according to Claim 1 or 2, in which the projecting portions are formed by convex, in particular part-spherical bosses (28) which project from an inwardly directed side (26) of each side part (24).

4. The padding element (10) according to one of the foregoing Claims, in which the padding part (16) is surrounded by a water-impermeable sheath (18).

5. The padding element (10) according to Claim 4, **characterized in that** at least the outer surface of the sheath (18) is electrically conductive.

6. The padding element (10) according to one of the foregoing Claims, in which the padding part (16) comprises a lower layer (20) of EPDM foam and an upper layer (22) of soft foam.

7. The padding element (10) according to one of the foregoing Claims, **characterized in that** the padding part (16) has a T-shaped cross section, the T-foot of which lies between the side parts (14) and the T-crosspieces of which cover the side parts (14).

8. The padding element (10) according to one of the foregoing Claims, in which the baseplate (12) is made of a material permeable to X-rays, in particular of plastic.

9. The padding element (10) according to one of the foregoing Claims, which is suitable for being arranged on a segment of a bed of an operating table which segment is connected in an articulated manner to a further segment of the bed.

10. The padding element (10) according to Claim 9, in which a shaped portion (30) is formed on at least one of the longitudinal ends of at least one of the side parts (14), such that the said shaped portion at least partially covers a joint between the said segments.

11. An operating table with a bed which comprises a plurality of segments adjustable in relation to one another, **characterized in that** an padding element (10) according to one of the foregoing Claims is arranged on at least one of the segments.

## Revendications

1. Elément de rembourrage (10) pour surface porteuse pour patient, ledit élément de rembourrage comportant
une plaque de fond (12), **caractérisé par**
deux parties latérales allongées (14) en matière alvéolaire indéformable (22) qui sont disposées sur cette plaque de fond (12) le long de deux bords opposés de celle-ci,
et une partie de rembourrage (16) qui est au moins partiellement en matière alvéolaire souple et qui peut être placée de façon amovible sur la plaque de fond (12) entre les parties latérales allongées (14) de façon à porter au niveau de ses côtés extérieurs (32) au moins partiellement contre chacune des parties latérales (14) adjacentes,
au moins une portion saillante (28) étant conformée au moins au niveau d'une partie latérale (14) de façon à s'engager dans un évidement (34) associé ménagé du côté extérieur (32) de la partie de rembourrage (16) lorsque la partie de rembourrage (16) est disposée entre les parties latérales (14).

2. Elément de rembourrage (10) selon la revendication 1, dans lequel les parties latérales sont en mousse structurée PUR.

3. Elément de rembourrage (10) selon la revendication 1 ou 2, dans lequel les portions saillantes sont formées par des boutons (28) convexes, notamment partiellement sphériques, qui font saillie depuis un côté (26), dirigé vers l'intérieur, de chacune des parties latérales (24).

4. Elément de rembourrage (10) selon l'une des revendications précédentes, dans lequel la partie de rembourrage (16) est entourée d'une enveloppe imperméable à l'eau (18).

5. Elément de rembourrage (10) selon la revendication 4, **caractérisé en ce que** au moins la surface extérieure de l'enveloppe (18) est électriquement conductrice.

6. Elément de rembourrage (10) selon l'une des revendications précédentes, dans lequel la partie de rembourrage (16) comporte une couche inférieure (20) en matière alvéolaire EPDM et une couche supérieure (22) en matière alvéolaire souple.

7. Elément de rembourrage (10) selon l'une des revendications précédentes, **caractérisé en ce que** la partie de rembourrage (16) a une section en forme de T dont le pied est situé entre les parties latérales (14) et la barre transversale coiffe les parties latérales (14).

8. Elément de rembourrage (10) selon l'une des revendications précédentes, dans lequel la plaque de fond (12) est en un matériau transparent aux rayons X, notamment en matière synthétique.

9. Elément de rembourrage (10) selon l'une des revendications précédentes, lequel élément de rembourrage est approprié pour être disposé sur un segment d'une surface porteuse d'une table d'opération et est relié de façon articulée à un autre segment de la surface porteuse.

10. Elément de rembourrage (10) selon la revendication 9, dans lequel une portion moulée (30) est conformée au niveau d'au moins une des parties latérales (14) au niveau d'une des extrémités longitudinales de façon à coiffer au moins partiellement une articulation entre lesdits segments.

11. Table d'opération dotée d'une surface porteuse qui comporte plusieurs segments réglables l'un par rapport à l'autre, **caractérisée en ce qu'**un élément de rembourrage (10) selon l'une des revendications précédentes est disposé sur au moins un des segments.
